# EUROPÄISCHE PATENTSCHRIFT

(11) **EP 0 823 237 B1**
(45) Veröffentlichungstag und Bekanntmachung des Hinweises auf die Patenterteilung: **09.10.2002**
(21) Anmeldenummer: 97112383.1
(22) Anmeldetag: 18.07.1997
(51) Int. Cl.: A61B 3/113

(54) **Videomessung der Position oder der Bewegung von Augen**
Video measurement of the position or movement of eyes
Mesure vidéo de la position ou du mouvement des yeux

(30) Priorität: 09.08.1996 DE 19632237
(43) Veröffentlichungstag der Anmeldung: 11.02.1998
(73) Patentinhaber: Erich Jaeger GmbH, 97204 Höchberg (DE)
(72) Erfinder: Kingma, Herman, Dr., 6229 VS Maastricht (NL)
(74) Vertreter: Hertz, Oliver, Dr.

(56) Entgegenhaltungen:
- DE-A- 3 206 530
- DE-U- 29 603 944
- US-A- 5 345 281

## Beschreibung

Die vorliegende Erfindung betrifft eine Anordnung zur Erfassung der Position und/oder der Bewegung von Augen, insbesondere mittels eines Video-Meßsystems.

Das menschliche Auge ist in der Lage, horizontale oder vertikale Bewegungen sowie Torsionsbewegungen auszuführen. Die Beobachtung, Erfassung und Messung dieser Bewegungen spielt eine wichtige Rolle bei der Beschreibung von motorischen Reaktionen, zum Beispiel in der Neurologie oder der Neuro-Ophthalmologie.

Es sind verschiedene Anordnungen zur Erfassung von Augenpositionen oder -bewegungen (Augenstellung) bekannt, die generell nach dem folgenden Prinzip arbeiten. Die Augen werden mit Infrarotlicht beleuchtet und das von den Augen reflektierte Licht wird von infrarotempfindlichen Detektorsystemen (z.B. Videokameras mit vorgeschalteten Infrarotfiltern) erfaßt. Durch sogenannte "Framegrabber" wird die Bildinformation verarbeitet und an eine Auswerteeinheit weitergegeben. In der Infrarotaufnahme ist die Pupille des Auges als nichtreflektierender Bereich zu der umgebenden Iris hin scharf abgegrenzt. Dies ergibt ein Bildmerkmal, mit dem die horizontale und vertikale Augenbewegung einfach bestimmt werden kann. Zur Erfassung der Torsionsbewegung eines Auges wird die Bildstruktur der Iris beobachtet. Die Auswertung dieser Bildstruktur ist erschwert, da diese insbesondere bei blauen Augen im Infrarotlicht kontrastarm ist. Weitere Störungen bei der Bildaufnahme ergeben sich aus Reflexionen der Infrarotbeleuchtung im Auge und durch im Blickfeld störende Wimpern.

Bei den bekannten Systemen zur Erfassung der Augenstellung werden geschlossene Systeme, bei denen die Detektoren in Blickrichtung vor dem Auge angeordnet sind, und offene Systeme unterschieden, bei denen sich in Blickrichtung des Auges lediglich ein Infrarotreflektor befindet, der die Infrarotreflexion vom Auge zum Detektor umlenkt.

Ein geschlossenes System ist zum Beispiel aus EP-A 0 456 166 bekannt. Eine brillenartige Anordnung weist in Blickrichtung jedes Auges eine Videokamera auf, außerhalb deren Umfangsberandung Infrarot-LED's angeordnet sind. Das von den LED's ausgehende Licht wird von jedem Augenbulbus reflektiert und auf die Videokameras gelenkt. Zur Verminderung von störenden Reflexionen zum Beispiel aus dem Augeninneren sind bei diesem System Polarisationsfilter vorgesehen.

Geschlossene Systeme haben den generellen Nachteil eines geringen Abstandes zwischen dem Detektorsystem und dem Augenbulbus. Sollen zur Winkelbestimmung Strukturmerkmale der Iris erfaßt werden, so müssen diese im Detektorsystem scharf abgebildet werden. Dies erfordert wegen des bei dem geringen Bulbus-Detektor-Abstand geringen Tiefenschärfebereiches eine häufige Korrektur der Abbildungsschärfe.

Weitere geschlossene Systeme werden in WO 94/16612 und in WO 95/28879 beschrieben. Geschlossene Systeme haben neben den oben genannten Problemen den Nachteil, daß eine Erfassung der Augenposition oder -bewegung unter optischer Stimulierung nicht möglich ist, da die Detektoren das Augenblickfeld verdecken.

Demgegenüber stellen offene Systeme eine Verbesserung dar, da der genannte Infrarotreflektor in Blickrichtung des Auges für sichtbares Licht durchlässig ist und somit eine optische Stimulierung des Auges erlaubt. So ist allgemein ein offenes System zur Erfassung von Augenbewegungen bekannt, das die folgenden Merkmale aufweist. Ein auf dem Stirnschädelbereich bzw. den Ohren aufsitzender Träger haltert jeweils für jedes Auge einen in der Blickrichtung des Auges schwenkbar angeordneten Infrarotreflektor und ein in Bezug auf die Gesichtschädelfläche seitlich angeordnetes Beleuchtungs- und Detektorsystem. Die Beleuchtungs- und Detektorsysteme sind seitlich neben der Gesichtschädelfläche, jedoch in Blickrichtung vor dieser angeordnet, um Abschattungen durch Augenbrauen, Teile der Schläfe oder dergl. zu vermeiden.

Weitere ähnlich aufgebaute, offene Systeme sind aus US-A-4 702 575 und US-A-5 345 281 bekannt. Die bekannten Augenstellungs-Erfassungssysteme vom offenen Typ besitzen die folgenden Nachteile. Die Infrarotreflektoren sind zur Gewährleistung der Schwenkbarkeit (Justierbarkeit) in ihrer Größe beschränkt. Daraus ergibt sich, daß für die Messung nur ein eingeschränktes Gesichtsfeld bzw. ein eingeschränktes Blickfeld berücksichtigt werden kann. Sobald ein interessierendes Bildmerkmal durch eine Augenbewegung über den Rand des Infrarotreflektors hinausläuft, ist die Augenstellungserfassung nicht mehr möglich. Ein weiterer Nachteil des bekannten offenen Systems besteht darin, daß der Bulbus-Detektor-Abstand gegenüber dem geschlossenen System zwar erhöht ist, dies jedoch wegen der Anbringung des Detektorsystems seitlich vor der Gesichtschädelfläche nur um einen Faktor von rund 2 bis 3. Damit ist nur eine geringe Verbesserung der Tiefenschärfe erzielbar. Schließlich besitzt das bekannte offene System eine geringe Einstellstabilität der optischen Komponenten. Durch unachtsame Bewegungen der Untersuchungsperson (z.B. Greifen ins Gesicht, Stirnrunzeln) werden Dejustierungen verursacht, was zu einer aufwendigeren Messung führt.

Aufgabe der vorliegenden Erfindung ist es, eine verbesserte Anordnung zur Erfassung von Augenpositionen und/oder -bewegungen bereitzustellen, die zuverlässiger arbeitet und Beschränkungen der bekannten Systeme überwindet.

Diese Aufgabe wird durch die Anordnung mit den Merkmalen gemäß Patentanspruch 1 gelöst. Vorteilhafte Ausführungsformen ergeben sich aus den abhängigen Patentansprüchen.

Die Erfindung geht von einem System zur Augenbeobachtung vom offenen Typ aus, das Beleuchtungsmittel zur Beleuchtung mindestens eines Augenbulbus, Detektormittel zur Erfassung von durch den Augenbulbus reflektiertem Licht (Erfassung einer Abbildung des Augenbulbus) und ein im Blickfeld des Auges angeordnetes Umlenkmittel mit einem ersten Reflektorbereich zur Umlenkung des vom Augenbulbus reflektierten Lichts auf das Detektormittel aufweist. Das Beleuchtungsmittel wird durch eine Infrarot-Beleuchtung gebildet. Diese kann zum Beispiel Infrarot-LED's oder einen Infrarotlaser umfassen. Das Detektormittel ist seitlich außerhalb des Blickfeldes des Auges angeordnet. Es wird durch einen infrarotempfindlichen Detektor (z.B. eine Videokamera) gebildet. Das Umlenkmittel ist ein Infrarotreflektor, der im Blickfeld des Auges angeordnet und dessen Oberfläche in Bezug auf die Blickrichtung des Auges einen Winkel ungleich 90° aufweist.

Die Erfindung löst die oben angegebene Aufgabe in überraschender Weise durch eine Maßnahme, mit der sowohl die mechanischen Stabilitätseigenschaften als auch die optischen Eigenschaften eines Systems zur Augenbeobachtung im Vergleich zu den herkömmlichen Systemen in entscheidender Weise verbessert werden. Diese Maßnahme besteht darin, daß das Umlenkmittel mindestens einen weiteren Reflektorbereich besitzt und so ausgelegt ist, daß das vom Augenbulbus reflektierte Infrarotlicht vor Erreichen des Detektormittels einer Mehrfach-Reflexion unterliegt. Somit kann jedes Detektormittel seitlich außerhalb der Sehachse eines Auges auf der dem untersuchten Auge entgegengesetzten Gesichtsseite angeordnet werden. Das Umlenkmittel wird in Bezug auf die Blickrichtung des Auges so angeordnet, daß das vom Augenbulbus reflektierte Licht die Blickrichtung des jeweils anderen Auges kreuzt.

Durch die Mehrfach-Reflexion des vom Augenbulbus reflektierten Lichts wird eine erhebliche Verlängerung des Meßlichtweges erzielt. Simultan erlaubt diese Maßnahnme eine ortsfeste Anordnung des Umlenkmittels an dem erfindungsgemäßen Augenbeobachtungssystem, wodurch die Stabilität erhöht wird. Schließlich kann das Umlenkmittel so gestaltet werden, daß keinerlei Beschränkung des Blickfeldes auftritt.

Das Umlenkmittel wird vorzugsweise so ausgelegt, daß das vom Augenbulbus reflektierte Licht vor Erfassung durch die Detektormittel einer Zweifach-Reflexion unterliegt. Eine erste Reflexion findet auf dem in Blickrichtung vor dem untersuchten Auge befindlichen Teil des Umlenkmittels statt. Eine zweite Reflexion erfolgt auf dem in Blickrichtung vor dem jeweils anderen Auge befindlichen Teil des Umlenkmittels. Durch geeignete Dimensionierung des Umlenkmittels sind die Längen der Lichtwege vom Augenbulbus zum Ort der ersten Reflexion bzw. vom Ort der ersten Reflexion zum Ort der zweiten Reflexion bzw. vom Ort der zweiten Reflexion zum Detektormittel im wesentlichen etwa gleich groß. Dadurch wird erreicht, daß trotz des großen Lichtweges bei Verfolgung einer Augenbewegung an dem Detektormittel eine nur geringe Auslenkung des einfallenden Lichtes stattfindet. Somit können auch bei dem erfindungsgemäßen Augenbeobachtungssystem die üblichen miniaturisierten Videokameras eingesetzt werden.

Gemäß einer besonderen Ausführungsform der Erfindung wird ein Augenbeobachtungssytem in Gestalt einer Brille angegeben, das hinter den Ohren und am Nasenrücken der Untersuchungsperson durch Drei-Punkt-Halterung stabil aufsitzt. Dieses System ist zur simultanen Beobachtung beider Augenbulbi durch mehrere Beleuchtungs- und Detektormittel (zwei Videokameras) vorgesehen, wobei jeweils eine Kamera neben einer Seite des Gesichts einen Augenbulbus der entgegengesetzten Seite des Gesichts erfaßt.

Ausführungsformen des erfindungsgemäßen Augenbeobachtungssystems werden im folgenden unter Bezug auf die beigefügte Figuren beschrieben. Es zeigen:
- Figur 1:: eine Draufsicht auf ein erstes Ausführungsbeispiel der Erfindung;
- Figur 2:: eine Draufsicht auf ein zweites Ausführungsbeispiel der Erfindung.
- Figur 3:: eine Frontalansicht auf das in Fig. 2 gezeigte System.

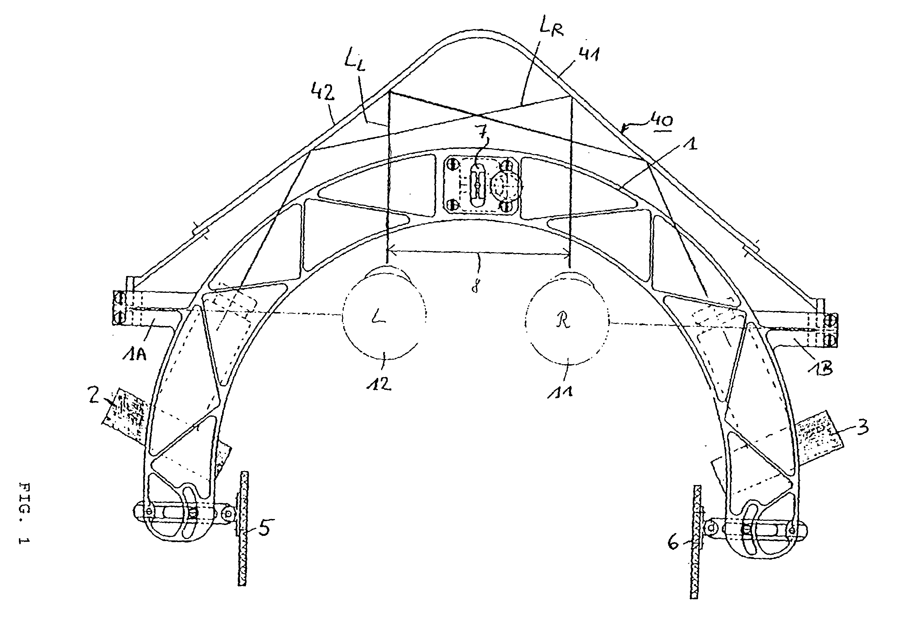

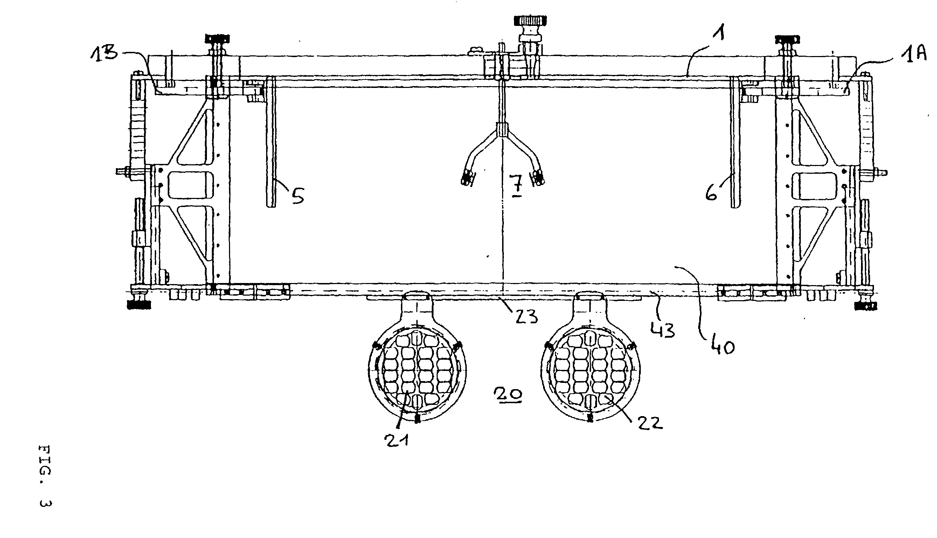

Ein erfindungsgemäßes System zur Augenbeobachtung umfaßt einen tragenden Rahmen 1, an dem das Beleuchtungsmittel 20 (in Fig. 1 nicht gezeigt), die Videokameras 2, 3 (in Fig. 3 nicht gezeigt) und das Umlenkmittel 40 angebracht sind. Der Rahmen 1 ist in Leichtbauweise ausgeführt. Die dargstellte Struktur kann aus einem bogenförmigen Teil aus leichtem und stabilem Material (z.B. Aluminium, Titan oder dergl.) herausgefräst sein. Der Rahmen besitzt eine leichte und in alle Richtungen verwindungssteife Gestellstruktur. Er umfaßt insbesondere Stabilisierungsstreben und einen Boden mit einer Dicke von rund 1 mm. Der Rahmen 1 besitzt drei Auflageelemente 5, 6 und 7. Diese entsprechen den Auflagepunkten einer Brille. Die Auflageelemente 5 und 6 liegen hinter den Ohren und das Auflageelement 7 auf dem Nasenrücken auf. Die Drei-Punkt-Auflage (5, 6, 7) gewährleistet einen sicheren Sitz. Verschiebungen durch Muskelbewegungen (z.B. Stirnrunzeln) werden ausgeschlossen. Damit bleibt vorteilhafterweise die vor der Messung einmal eingestellte Justierung des Systems in Bezug auf den Kopf (und somit die Augen) der Untersuchungsperson erhalten.

Am Boden des Rahmens 1 sind die beiden Videokameras 2, 3 schwenkbar befestigt. Durch geeignetes Verschwenken der Kameras wird deren Aufnahmeposition auf den Augenabstand 8 (siehe Fig. 1) Untersuchungsperson abgestimmt. Diese Verschwenkung beträgt normalerweise nur wenige Grad.

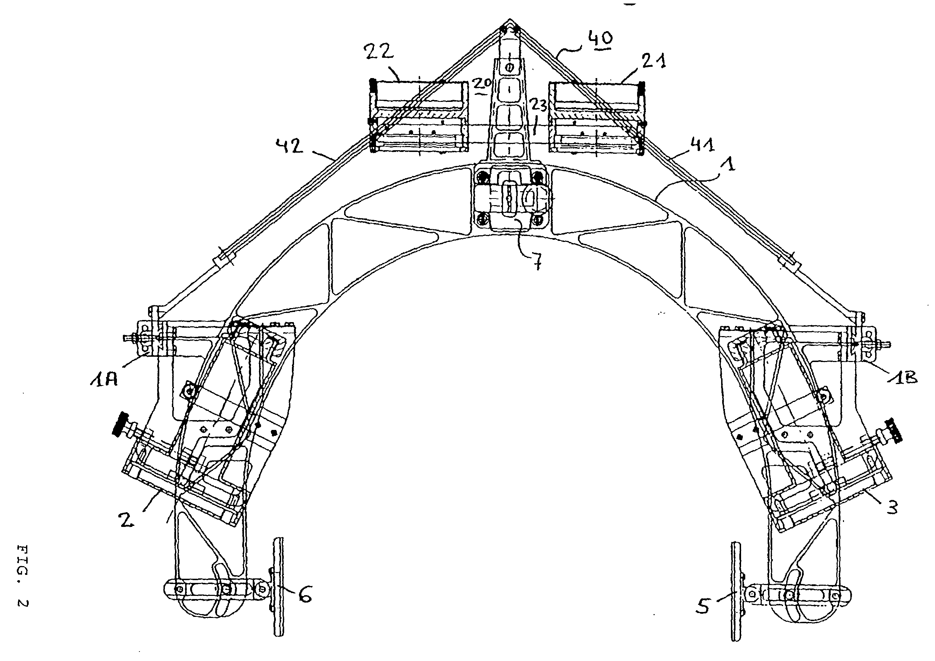

Der Rahmen 1 besitzt auf seiner äußeren Seite zwischen den Auflageelementen 5 und 7 bzw. 6 und 7 jeweils einen Vorsprung 1A bzw. 1B. Über die Vorsprünge 1A und 1B ist das Umlenkmittel 40 an dem Rahmen 1 befestigt. Das Umlenkmittel 40 besteht aus einem Reflektorelement 41, 42, das abgesehen von der Halterung an den Ansätzen 1A und 1B freitragend in der Betriebsposition derart oberhalb des Rahmens 1 angeordnet ist, daß das Blickfeld der Untersuchungsperson in horizontaler Richtung im wesentlichen vollständig und in vertikaler Richtung mindestens 80% abgedeckt ist. Der Infrarotreflektor des Umlenkmittels 40 kann, wie in Fig. 1 dargestellt, aus einem gebogenen Kunststoffteil bestehen. Ersatzweise ist es möglich, zwei Kunststoff- oder Glasplatten 41, 42 unter einem geeigneten Winkel zu verbinden (z. B. Verkleben) wie es in Fig. 2 dargestellt ist. Der Winkel zwischen beiden Reflektorhälften beträgt vorzugsweise 100°. Außerhalb des Verbindungsbereiches in der Mitte des Reflektors sind die Reflektoroberflächen vorzugsweise eben. Für eine optimale, verzerrungsfreie Bildwiedergabe besitzen die Reflektoroberflächen auf der der Untersuchungsperson zugewandten Seite vorzugsweise die Eigenschaften von optisch ebenen Platten. Die ebenen Innenseiten werden mit einer infrarot-reflektierenden Schicht versehen.

Der Reflektor des Umlenkmittels 40 ist vorzugsweise für sichtbares Licht durchlässig und transparent. Es sind jedoch auch Ausführungsformen möglich, bei denen der Reflektor aus einem diffus streuenden oder undurchlässigen Material oder einem Material mit Filtereigenschaften im optischen Bereich besteht. Gemäß einer bsonderen Ausführungsform der Erfindung sind die Ansätze 1A und 1B mit einer lösbaren Verbindung (z.B. (Rast-Verbindung) versehen, um einen Austausch des Umlenkmittels je nach den Untersuchungsanforderungen zu ermöglichen.

In Fig. 1 ist ein optischer Weg L_{L}, L_{R} des Lichtes vom linken bzw. rechten Auge 12, 11 über das Umlenkmittel 40 zu den Videokameras 3 bzw. 2 beispielhaft dargestellt. Das vom (nicht gezeigten) Beleuchtungsmittel ausgehende Licht wird vom Bulbus 12 reflektiert und entlang des Lichtweges L_{L} zu dem in der Figur linken Teil des Umlenkmittels 4 geführt, von dort die Blickrichtung des rechten Bulbus 11 kreuzend auf den rechten Teil des Umlenkmittels 40 gerichtet und schließlich von dort auf die Videokamera 3 gelenkt. Für das vom rechten Bulbus 11 reflektierte Licht gilt entsprechend der Lichtweg L_{R}.

In den Fig. 2 und 3 ist die Anbringung des Beleuchtungsmittels 20 dargestellt. Das Beleuchtungsmittel 20 besteht aus zwei Beleuchtungseinheiten 21, 22, die jeweils einem Auge zugeordnet sind. Die Beleuchtungseinheiten 21, 22 sind an dem Umlenkmittel 40 befestigt. Mit einer Tragevorrichtung sind sie in Betriebsposition unterhalb des Umlenkmittels 40 derart angebracht, daß jeder Bulbus direkt durch den Abstand zwischen der Unterkante 43 des Umlenkmittels 40 und dem Gesicht bestrahlbar ist. Jede Beleuchtungseinheit umfaßt Strahlungsquellen in Form von einzelnen Leuchtdioden, Leuchtdioden-Anordnungen (in Fig. 3 dargestellt) oder Laserdioden-Elementen. Die Tragevorrichtung 23 des Beleuchtungsmittels 20 kann dazu ausgelegt sein, daß jede Beleuchtungseinheit 21, 22 verstellbar angebracht ist. So kann beispielsweise der Beleuchtungswinkel von den Beleuchtungseinheiten 21, 22 jeweils auf einen Bulbus durch Verschwenken der Beleuchtungseinheiten veränderlich sein.

Zusätzlich zu den dargestellten Komponenten des erfindungsgemäßen Beobachtungssystems können Polarisationsfilter vorgesehen sein. Jeweils den Beleuchtungsmitteln 40 und den Kameras 2, 3 kann ein Polarisationsfilter vorgeschaltet sein. Die Filter werden relativ zueinander derart eingestellt, daß störende Reflexionen vom Bulbus minimiert werden. Dies ist insbesondere bei der Messung von rotatorischen Augenbewegungen (Drehbewegungen) von Vorteil, da die Reflexe vom Bulbus bei der Drehbewegung ortsstabil bleiben, während von Bildverarbeitungsmitteln die sich verschwenkende Struktur der Iris ausgewertet wird.

Die Beleuchtungs- und Detektormittel sind mit geeigneten (nicht dargestellten) elektrischen Leitungen versehen.

Zu den beschriebenen Ausführungsbeispielen der Erfindung sind die folgenden Modifikationen möglich:

Der brillenartige Rahmenaufbau kann durch eine andere geeignete Halterung ersetzt werden, die die erforderliche Stabilität gewährleistet. Das Umlenkmittel 40 kann prinzipiell so aufgebaut sein, daß weitere Reflexionen zur Verlängerung des Lichtweges auftreten. Es kann vorgesehen sein, daß der Winkel zwischen den Reflektorflächen des Umlenkmittels einstellbar ist.

Das Beleuchtungsmittel 20 kann an anderen geeigneten Positionen des Umlenkmittels oder des Rahmens 1 angebracht sein. Beispielsweise ist es möglich, das Beleuchtungsmittel in die Unterkante des Umlenkmittels zu integrieren oder in Betriebsposition oberhalb oder seitlich zu dem Umlenkmittel und dem Rahmen 1 anzubringen. Es kann für beide Augen eine gemeinsame Beleuchtungseinheit vorgesehen sein.

Ein Meßgerät zur Beobachtung, Erfassung bzw. Messung der Position und/oder der Bewegung von Augen umfaßt neben dem beschriebenen System eine Bedienungs- und Auswertungseinheit, die insbesondere herkömmliche Mittel zur Bildverarbeitung, zur Bilddarstellung und zur Quantifizierung von statischen oder dynamischen Bildmerkmalen enthält.

Das erfindungsgemäße System zur Beobachtung der Augenstellung besitzt die folgenden Vorteile:

Das erfindungsgemäße Prinzip der Mehrfach-Umlenkung führt zu einem vergrößerten Auge-Kamera-Abstand und somit zu einer erhöhten Tiefenschärfe. Damit wird die Erfassung von kontrastarmen Bildmustern, insbesondere der Struktur in der Iris, verbessert und die Zuverlässigkeit der Augenmessung erhöht.

Die Verbesserung der Tiefenschärfe erlaubt auch die Messung in einem größeren Meßbereich, da der gesamte Bulbus auch im Bereich der Krümmung scharf abgebildet wird.

Das Umlenkmittel ermöglicht ein weitgehend ungehindertes Sehfeld. Beschränkungen für die möglichen Augenbewegungen bei der Untersuchung sind minimiert.

Im Unterschied zu den bekannten offenen Systemen erlaubt es die erfindungsgemäße Anordnung, die Videokameras seitlich neben dem Kopf zu positionieren. Ein Überhängen der Kameramassen an einem Träger vor der Gesichtsfläche wird vermieden, so daß sich die Stabilität der Gesamtanordnung erhöht.

Die Justierung des erfindungsgemäßen Systems ist wesentlich vereinfacht, da für jedes Auge lediglich eine Videokamera verschwenkt werden muß. Das fixierte Umlenkmittel muß nicht justiert werden. Die Gefahr der Dejustierung durch Bewegungen der Untersuchungsperson ist minimiert.

## Patentansprüche

1. Anordnung zur Augenbeobachtung, die für jeden zu untersuchenden Augenbulbus umfaßt:
- Beleuchtungsmittel (20) zur Beleuchtung des Augenbulbus;
- Detektormittel (2, 3) zur Erfassung einer Abbildung des Augenbulbus, welche Detektormittel (2, 3) seitlich außerhalb der Sehachse des Auges angeordnet sind; und
- eine Spiegelanordnung (40) zur Umlenkung des vom Auge reflektierten Lichtes auf die Detektormittel (2, 3), welche Spiegelanordnung (40) einen ersten, in der Sehachse des Auges angeordneten Reflektorbereich (41) enthält,
**dadurch gekennzeichnet, daß**
die Spiegelanordnung (40) in Blickrichtung vor dem jeweils anderen Auge mindestens einen zweiten Reflektorbereich (42) enthält und so ausgelegt ist, daß das vom Auge (11, 12) zu dem Detektormittel gelenkte Licht einer ersten Reflexion am ersten Reflektorbereich und mindestens einer zweiten Reflexion am zweiten Reflektorbereich unterliegt.

2. Anordnung gemäß Anspruch 1, bei der die Reflektorbereiche (41, 42) durch zwei im wesentlichen ebene, jeweils in den Sehachsen der Augen angeordnete Reflektoren gebildet werden.

3. Anordnung gemäß Anspruch 2, bei der das Detektormittel zur Erfassung des rechten (11) bzw. linken (12) Auges auf der linken (2) bzw. rechten (3) Seite des Gesichts einer Untersuchungsperson angeordnet ist.

4. Anordnung gemäß einem der vorhergehenden Ansprüche, bei der das Beleuchtungsmittel (20), das Detektormittel (2, 3) und das Umlenkmittel (40) an einem Rahmen (1) angebracht sind, der Auflageelemente (5, 6, 7) zur brillenartigen Auflage an den Ohren und am Nasenrücken der Untersuchungsperson aufweist.

5. Anordnung gemäß Anspruch 4, bei der das Umlenkmittel (40) mit den Reflektoren ein winkliges Band bildet, das das Gesichtsfeld der Augen im wesentlichen überdeckt und an seinen Enden am Rahmen (1) angebracht ist.

6. Anordnung gemäß einem der vorhergehenden Ansprüche, bei der das Beleuchtungsmittel (20) durch infrarot-emittierende Elemente gebildet werden.

7. Anordnung gemäß einem der vorhergehenden Ansprüche, bei der das Detektormittel Videokameras (2, 3) umfaßt.

8. Anordnung gemäß einem der vorhergehenden Ansprüche, bei der das Beleuchtungsmittel (20) und das Detektormittel (2, 3) jeweils mit Polarisationsfiltern versehen ist.

## Claims

1. A system for observing the eyes which, for each eyeball to be examined, includes:
illuminating means (20) for illuminating the eyeball;
detector means (2, 3) for detecting an image of the eyeball, which detector means (2, 3) is arranged laterally outside the viewing axis of the eye; and
a mirror arrangement (40) for deflecting the light reflected from the eye onto the detector means (2, 3), which mirror arrangement (40) includes a first reflector region (41) arranged in the viewing axis of the eye,
**characterised in that**
the mirror arrangement (40) includes at least one second reflector region (42) in front of the other eye in the viewing direction and is so designed that the light deflected from the eye (11, 12) to the detector means is subjected to a first reflection at the first reflector region and at least one second reflection at the second reflector region.

2. A system as claimed in claim 1, in which the reflector regions (41, 42) are constituted by two substantially flat reflectors arranged respectively in the viewing axes of the eyes.

3. A system as claimed in claim 2, in which the detector means for detecting the right-hand (11) and left-hand (12) eye is arranged on the left-hand (2) and right-hand (3) side, respectively, of the face of the person to be examined.

4. A system as claimed in one of the preceding claims, in which the illuminating means (20), detector means (2, 3) and deflector means (40) are mounted on a frame (1), which has support elements (5, 6, 7) for support in the manner of spectacles on the ears and the bridge of the nose of the subject of the examination.

5. A system as claimed in claim 4, in which the deflecting means (40) together with the reflectors constitutes an angled strip, which substantially covers the field of view of the eyes and is mounted on the frame (1) at its ends.

6. A system as claimed in one of the preceding claims, in which the illuminating means (20) is constituted by infrared-emitting elements.

7. A system as claimed in one of the preceding claims, in which the detector means includes video cameras (2, 3).

8. A system as claimed in one of the preceding claims, in which the illuminating means (20) and the detector means (2, 3) are provided with respective polarisation filters.

## Revendications

1. Dispositif d'observation des yeux, qui comprend pour chaque globe oculaire à examiner :
- des moyens d'éclairage (20) pour éclairer le globe oculaire ;
- des moyens de détection (2, 3) pour détecter une reproduction du globe oculaire, lesquels moyens de détection (2, 3) sont disposés latéralement hors de l'axe de vision de l'oeil ; et
- un agencement de miroirs (40) pour dévier la lumière réfléchie par l'oeil sur les moyens de détection (2, 3), lequel agencement de miroirs (40) contient une première zone de réflexion (41) disposée dans l'axe de vision de l'oeil,
**caractérisé en ce que** l'agencement de miroirs (40) contient devant l'autre oeil, dans le sens d'observation, au moins une deuxième zone de réflexion (42) et est conçu de manière que la lumière dirigée par l'oeil (11, 12) vers le moyen de détection, soit soumise à une première réflexion sur la première zone de réflexion et à au moins une deuxième réflexion sur la deuxième zone de réflexion.

2. Dispositif selon la revendication 1 dans lequel les zones de réflexion (41, 42) sont formées par deux réflecteurs sensiblement plans, disposés chacun dans l'axe de vision des yeux.

3. Dispositif selon la revendication 2, dans lequel le moyen de détection pour détecter l'oeil droit (11) ou l'oeil gauche (12) est disposé respectivement sur le côté gauche (2) et le côté droit (3) du visage d'une personne à examiner.

4. Dispositif selon l'une des revendications précédentes, dans lequel le moyen d'éclairage (20), le moyen de détection (2, 3) et le moyen de déviation (40) sont placés sur un cadre (1) qui comporte des éléments de support (5, 6, 7) pour être supportés à la manière de lunettes par les oreilles et l'arête du nez de la personne à examiner.

5. Dispositif selon la revendication 4, dans lequel le moyen de déviation (40) forme avec les réflecteurs une bande coudée qui recouvre sensiblement le champ de vision des yeux et qui, à ses extrémités, est placée sur le cadre (1).

6. Dispositif selon l'une des revendications précédentes, dans lequel le moyen d'éclairage (20) est formé par des éléments émettant une lumière infrarouge.

7. Dispositif selon l'une des revendications précédentes, dans lequel le moyen de détection comprend des caméras vidéo (2, 3).

8. Dispositif selon l'une des revendications précédentes, dans lequel le moyen d'éclairage (20) et le moyen de détection (2, 3) sont pourvus chacun de filtres de polarisation.
